# EUROPEAN PATENT SPECIFICATION

(11) **EP 4 206 649 B1**
(45) Date of publication and mention of the grant of the patent: **06.11.2024**
(21) Application number: 21871365.9
(22) Date of filing: 15.09.2021
(51) Int. Cl.: G01N 35/10, G01N 33/02

(54) **MICROFLUIDIC DETECTION SYSTEM, CONTROL METHOD THEREFOR, AND REFRIGERATOR**
MIKROFLUIDISCHES DETEKTIONSSYSTEM, STEUERUNGSVERFAHREN DAFÜR UND KÜHLSCHRANK
SYSTÈME DE DÉTECTION MICROFLUIDIQUE, PROCÉDÉ DE COMMANDE ASSOCIÉ, ET RÉFRIGÉRATEUR

(30) Priority: 27.09.2020 CN 202011029746
(43) Date of publication of application: 05.07.2023
(73) Proprietor: Qingdao Haier Refrigerator Co., Ltd., Laoshan District Qingdao Shandong 266101 (CN); Haier Smart Home Co., Ltd., Qingdao, Shandong 266101 (CN)
(72) Inventor: ZHAO, Bintang, Qingdao, Shandong 266101 (CN); FEI, Bin, Qingdao, Shandong 266101 (CN); LIU, Haoquan, Qingdao, Shandong 266101 (CN)
(74) Representative: Ziebig Hengelhaupt Intellectual Property Attorneys Patentanwaltskanzlei PartGmbB
(86) International application number: PCT/CN2021/118504
(87) International publication number: WO 2022/062994

(56) References cited:
- CN-A- 108 801 929
- CN-A- 108 801 929
- CN-A- 109 420 531
- CN-A- 109 499 633
- CN-A- 109 499 634
- CN-U- 208 526 655
- CN-U- 209 570 508
- DE-U1- 202007 007 707
- US-A1- 2007 122 819
- US-A1- 2012 003 725
- US-A1- 2018 169 648

## Description

### FIELD OF THE INVENTION

The present invention relates to refrigerating and freezing technologies, and particularly to a control method for a microfluidic detection system, the microfluidic detection system, and a refrigerator.

### BACKGROUND OF THE INVENTION

With the improvement of the living standard of people, pesticide residues, viruses, nutritional elements or other aspects of some edible food materials are usually required to be detected in daily life, so as to qualitatively or quantitatively obtain the conditions of the food materials. For example, due to the pesticide abuse problem, fruits, vegetables and agricultural and sideline products purchased daily by people may have the problem of excessive pesticide residue content, and if the problem of excessive pesticide residue content of the foods cannot be found in time, great harm may be caused after people ingest the foods. For another example, currently advocated breast feeding is best feeding for infants only when breast milk has normal nutritional value, but in cases of diseases, medicine taking, surgery or other cases of the mother, the milk secreted by the mother may have reduced content of nutritional elements and even produce viruses, thereby affecting the growth and health of the infants.

Among detection methods, the method for detection by using a microfluidic biochip is rapid, the size is small, and the method is suitable for household use. In order to make sample introduction of the microfluidic biochip more accurate and easy to control, a driving apparatus may be used to drive a sample liquid into the microfluidic biochip. However, mixture between the sample liquid and a reagent in the microfluidic biochip is quite uneven, such that a reaction therebetween is not sufficient, thus affecting the accuracy of a detection result.

### BRIEF DESCRIPTION OF THE INVENTION

An object of a first aspect of the present invention is to overcome at least one of the drawbacks of the prior art, and to provide a control method for a microfluidic detection system capable of improving the uniformity of mixture between a sample liquid entering a microfluidic biochip and a reagent therein, so as to guarantee the accuracy of a detection result.

A further object of the first aspect of the present invention is to avoid the problem that a part of the sample liquid flows out from a sample inlet to cause contamination when a sample liquid driving apparatus performs a liquid pushing action.

Another object of the first aspect of the present invention is to further improve the accuracy of the detection result.

An object of a second aspect of the present invention is to provide a microfluidic detection system operating according to any one of the above-mentioned control methods.

An object of a third aspect of the present invention is to provide a refrigerator having a microfluidic detection system operating according to any one of the above-mentioned methods. The present invention is defined by the method of independent claim 1, the system of independent claim 10 and the refrigerator of independent claim 11. In the following, in case parts of the description and drawing referring to embodiments, which are not covered by the claims are not presented as embodiments of the invention, but as examples useful for understanding the invention.

In the control method according to the present application, the sample liquid driving apparatus is controlled to periodically and repeatedly execute the first liquid pushing and drawing operation including the first liquid pushing action and the first liquid drawing action after the sample liquid flows into the detection pool; since driving forces applied to the sample liquid by the first liquid pushing action and the first liquid drawing action have opposite directions, the sample liquid can be promoted to repeatedly flow back and forth in the microfluidic biochip, thereby facilitating mixture between the sample liquid and the reagent, improving the mixing effect of the sample liquid and the reagent, facilitating the full reaction between the sample liquid and the reagent, and improving the accuracy of the detection result.

Further, after the sample liquid enters the reaction pool, the sample liquid driving apparatus first executes a liquid drawing action (i.e., the third liquid drawing action), such that at least part of the sample liquid in the micro-channel connected between the reaction pool and the sample inlet flows into the reaction pool, thus forming the preset space margin in the micro-channel, the preset space margin being used for accommodating the sample liquid pushed out by the sample liquid driving apparatus when the sample liquid driving apparatus executes the second liquid pushing action. Thus, the problem of contamination due to a small quantity of the sample liquid being pushed out from the sample inlet when the sample liquid driving apparatus performs the second liquid pushing action can be avoided.

Further, in the present application, the quantity of the sample liquid entering the reaction pool is accurately controlled to be matched with the quantity of the reagent in the chip, thus avoiding the influence on the detection result caused by too much or too little sample liquid, and further improving the accuracy of the detection result.

Further, in the present application, the quantity of the sample liquid pushed out and the quantity of the sample liquid drawn in in one and the same liquid pushing and drawing operation are the same, thus avoiding that due to large error accumulation caused by executing the liquid pushing and drawing operation multiple times, the accuracy of the detection result is affected.

According to the following detailed description of specific embodiments of the present invention in conjunction with drawings, those skilled in the art will better understand the aforementioned and other objects, advantages and features of the present invention.

### BRIEF DESCRIPTION OF THE DRAWINGS

Some specific embodiments of the present invention will be described below in detail in an exemplary rather than restrictive manner with reference to the drawings. Identical reference numerals in the drawings represent identical or similar components or parts. Those skilled in the art should understand that these drawings are not necessarily drawn to scale. In the drawings:
Fig. 1 is a schematic structural diagram of a microfluidic detection system according to an embodiment of the present invention;
Fig. 2 is a schematic sectional diagram of a microfluidic biochip in an embodiment of the present invention;
Fig. 3 is a schematic flow diagram of a control method for a microfluidic detection system according to a first embodiment of the present invention;
Fig. 4 is a schematic flow diagram of a control method for a microfluidic detection system according to a second embodiment of the present invention;
Fig. 5 is a schematic flow diagram of a control method for a microfluidic detection system according to a third embodiment of the present invention;
Fig. 6 is a schematic flow diagram of a control method for a microfluidic detection system according to a fourth embodiment of the present invention as defined in independent claim 1;
Fig. 7 is a schematic flow diagram of a control method for a microfluidic detection system according to a fifth embodiment of the present invention;
Fig. 8 is a schematic flow diagram of detection of a detection pool by a detection mechanism in an embodiment of the present invention;
Fig. 9 is a schematic flow diagram of detection of a detection pool by a detection mechanism in another embodiment of the present invention;
Fig. 10 is a schematic flow diagram of controlling a sample liquid driving apparatus to periodically and repeatedly execute a first liquid pushing and drawing operation in an embodiment of the present invention;
Fig. 11 is a schematic flow diagram of a sample loading process of a microfluidic detection system according to an embodiment of the present invention; and
Fig. 12 is a schematic structural diagram of a refrigerator according to an embodiment of the present invention.

### DETAILED DESCRIPTION

The present application firstly provides a control method for a microfluidic detection system, which is used for qualitatively or quantitatively detecting a preset detection parameter of a sample liquid. The preset detection parameter may be, for example, a pesticide residue parameter for indicating whether a pesticide residue content exceeds the standard and/or a specific value of the pesticide residue content, a nutrient parameter for indicating whether a nutritional element meets the standard and/or a specific content of the nutritional element, a specific substance parameter for indicating whether a specific harmful substance (for example, a specific virus) exceeds the standard and/or a specific content thereof, or the like.

The control method for a microfluidic detection system can be generally divided into two parts of sample loading and detection. First, the detection part is specifically described in the present application.

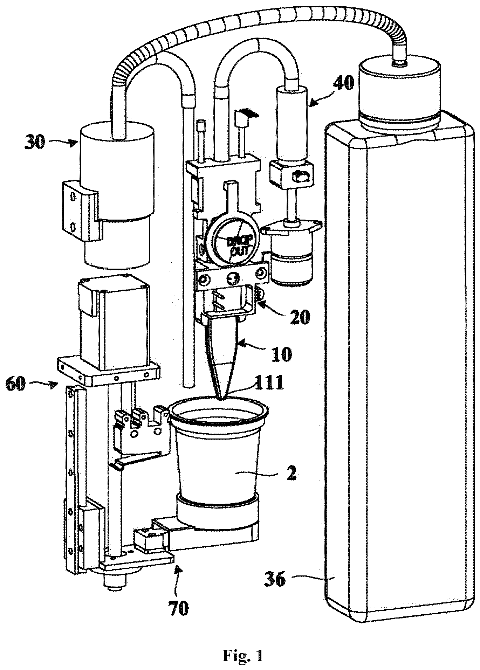

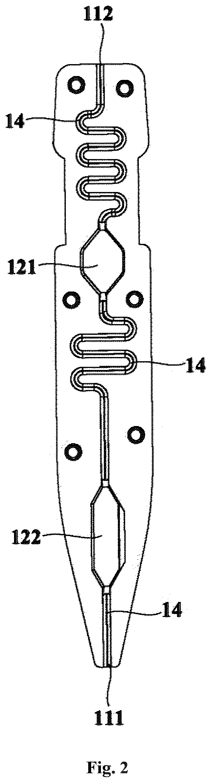

Fig. 1 is a schematic structural diagram of the microfluidic detection system according to an embodiment of the present invention, and for ease of understanding, a sample cup 2 is also shown in Fig. 1. The microfluidic detection system 1 according to the present invention includes a microfluidic biochip 10, a sample liquid driving apparatus 40 and a detection mechanism 20. Fig. 2 is a schematic sectional diagram of the microfluidic biochip in an embodiment of the present invention. The microfluidic biochip 10 is provided with a sample inlet 111 for receiving a sample liquid, a communication port 112 communicated with the sample liquid driving apparatus 40 and a detection pool 121 formed inside the microfluidic biochip 121, the detection pool 121 is configured to hole a detection reagent, and the sample inlet 111, the detection pool 121 and the communication port 112 are communicated in sequence.

The applicant recognizes that in practical applications, the microfluidic biochip 10 is usually fixed, the sample liquid driving apparatus 40 and the microfluidic biochip 10 are usually communicated hermetically, and when the microfluidic detection system 1 is integrated on a refrigerator, the microfluidic biochip 10 is preferably vertically disposed with the sample inlet at the bottom thereof, such that since the oscillation amplitude of the microfluidic biochip 10 is quite limited, it is not suitable to promote uniform mixture between the sample liquid and the reagent by means of oscillation, and the oscillation process thereof may cause a series of problems, such as air leakage at the connection between the sample liquid driving apparatus 40 and the microfluidic biochip 10, liquid leakage at the sample inlet 111, or the like.

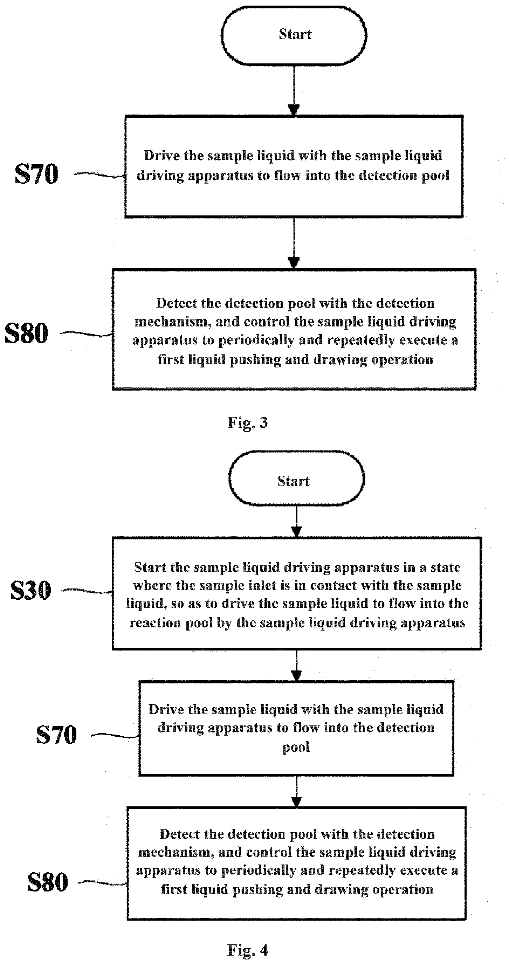

For this reason, the present application provides a control method for a microfluidic detection system; Fig. 3 is a schematic flow diagram of the control method for a microfluidic detection system according to a first embodiment of the present invention, and referring to Fig. 3, the control method according to the present invention includes:
step S70: driving the sample liquid with the sample liquid driving apparatus 40 to flow into the detection pool 121; and
step S80: detecting the detection pool 121 with the detection mechanism 20, and controlling the sample liquid driving apparatus 40 to periodically and repeatedly execute a first liquid pushing and drawing operation, the first liquid pushing and drawing operation including a first liquid pushing action used for prompting the sample liquid to flow in the direction of the sample inlet 111, and a first liquid drawing action used for prompting the sample liquid to flow in the direction of the detection pool 121.

In the microfluidic detection system according to the present invention, the sample liquid is driven to automatically flow into the microfluidic biochip by the sample liquid driving apparatus, a manual operation of a user is avoided, and accurate control is facilitated. In the control method according to the present application, the sample liquid driving apparatus 40 is controlled to periodically and repeatedly execute the first liquid pushing and drawing operation including the first liquid pushing action and the first liquid drawing action after the sample liquid flows into the detection pool 121; since driving forces applied to the sample liquid by the first liquid pushing action and the first liquid drawing action have opposite directions, the sample liquid can be promoted to repeatedly flow back and forth in the microfluidic biochip 10, thereby facilitating mixture between the sample liquid and the detection reagent, improving the mixing effect of the sample liquid and the detection reagent, facilitating the full reaction between the sample liquid and the detection reagent, and improving the accuracy of the detection result.

Further, in one and the same first liquid pushing and drawing operation, the quantity of the sample liquid pushed out by the sample liquid driving apparatus 40 performing the first liquid pushing action is the same as the quantity of the sample liquid drawn in by the sample liquid driving apparatus performing the first liquid drawing action. Thus, the final quantity of the sample liquid in the detection pool 121 can be guaranteed to be kept unchanged, and it is avoided that due to large error accumulation caused by executing the first liquid pushing and drawing operation multiple times, the accuracy of the detection result is affected.

In some embodiments, the microfluidic biochip 10 is further provided with a reaction pool 122 connected between the detection pool 121 and the sample inlet 111, and the sample inlet 111, the reaction pool 122, the detection pool 121 and the communication port 112 are sequentially communicated through a micro-channel 14 to form a main channel. For a specific sample liquid or for some specific detection parameters of the sample liquid, the sample liquid may be required to react with a reaction reagent first and then react with the detection reagent, and the detection mechanism detects a solution after the final reaction to obtain a preset detection parameter of the specific sample liquid, thus avoiding a reaction or mutual influence between the reaction reagent and the detection reagent, and improving the accuracy of the detection result. For example, when the microfluidic detection system 1 is required to be used to detect a pesticide residue parameter of the sample liquid, an enzyme inhibition rate method is preferred, and since a pesticide residue content is qualitatively detected in the method, the detection speed is higher, and the method is more suitable for household use. At this point, the reaction reagent and the detection reagent for the microfluidic biochip 10 may be an enzyme reagent and a color developing agent respectively. The reaction pool 122 is configured to allow the sample liquid to react with the enzyme reagent therein, and the sample liquid after the reaction with the enzyme reagent flows into the detection pool 121 to react with the color developing agent in the detection pool 121.

Fig. 4 is a schematic flow diagram of the control method for a microfluidic detection system according to a second embodiment of the present invention; referring to Fig. 4, in these embodiments, before the step S70 of driving the sample liquid to flow into the detection pool 121, the control method according to the present invention further includes:
step S30: starting the sample liquid driving apparatus 40 in a state where the sample inlet 111 is in contact with the sample liquid, so as to drive the sample liquid to flow into the reaction pool 122 by the sample liquid driving apparatus 40.

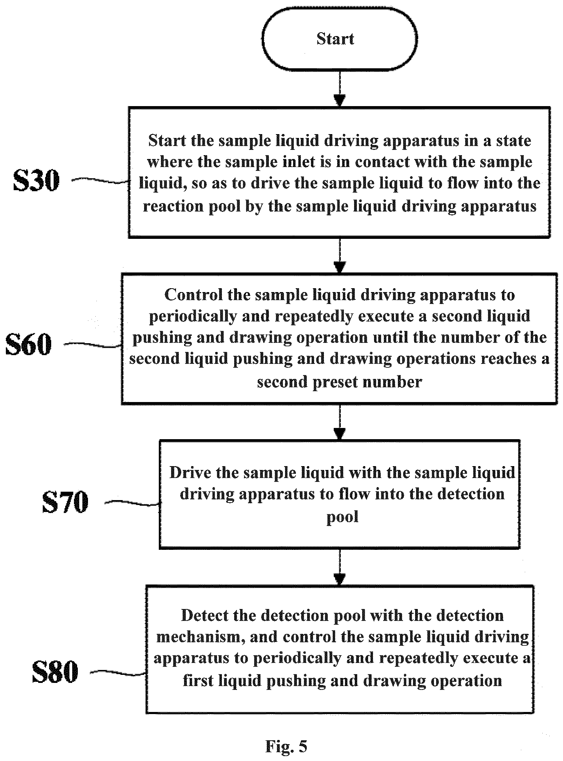

Fig. 5 is a schematic flow diagram of the control method for a microfluidic detection system according to a third embodiment of the present invention. Referring to Fig. 5, further, in order to improve the effect of mixture between the sample liquid and the reaction reagent, in some embodiments, after the step S30 of driving the sample liquid to flow into the reaction pool 122 and before the step S70 of driving the sample liquid to flow into the detection pool 121, the control method according to the present invention further includes:
step S60: controlling the sample liquid driving apparatus 40 to periodically and repeatedly execute a second liquid pushing and drawing operation until the number of the second liquid pushing and drawing operations reaches a second preset number. The second liquid pushing and drawing operation includes a second liquid pushing action for promoting the sample liquid to flow towards the sample inlet 111 and a second liquid drawing action for promoting the sample liquid to flow towards the reaction pool 122. The second preset number is a preset number for enabling the sample liquid and the reaction reagent to be uniformly mixed according to experimental verification.

Since driving forces applied to the sample liquid by the second liquid pushing action and the second liquid drawing action have opposite directions, the sample liquid can be promoted to repeatedly flow back and forth in the microfluidic biochip 10, thereby facilitating the mixture between the sample liquid and the reaction reagent, improving the mixing effect of the sample liquid and the reaction reagent, and facilitating the full action between the sample liquid and the reaction reagent.

Further, in one and the same second liquid pushing and drawing operation, the quantity of the sample liquid pushed out by the sample liquid driving apparatus 40 performing the second liquid pushing action is the same as the quantity of the sample liquid drawn in by the sample liquid driving apparatus performing the second liquid drawing action. Thus, the final quantity of the sample liquid in the reaction pool 122 can be guaranteed to be kept unchanged, and it is avoided that due to large error accumulation caused by executing the second liquid pushing and drawing operation multiple times, the quantity of the sample liquid flowing to the detection pool 121 is affected and thus the accuracy of the detection result is affected.

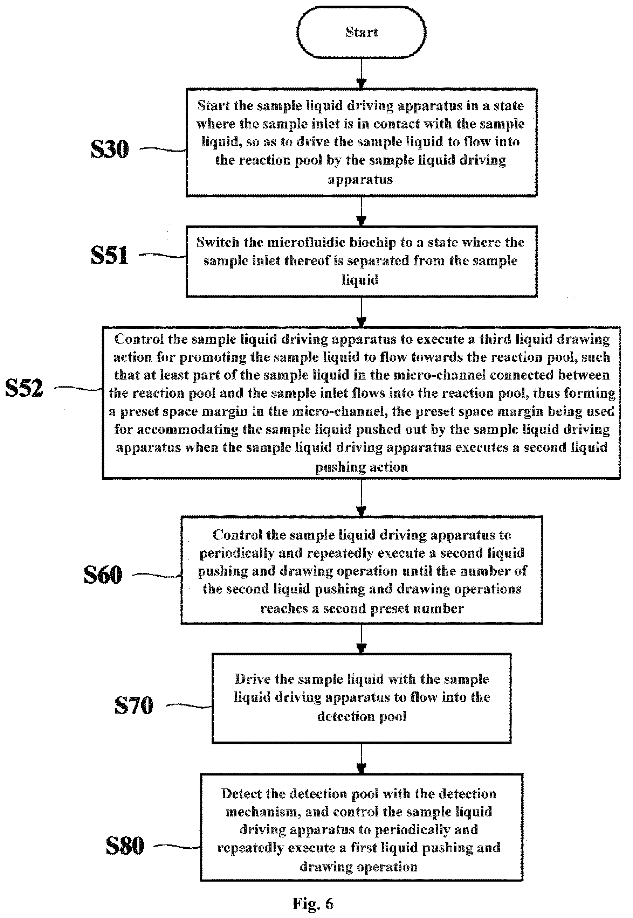

Fig. 6 is a schematic flow diagram of the control method for a microfluidic detection system according to a fourth embodiment of the present invention, as also defined in independent claim 1. Referring to Fig. 6, in some embodiments, after the step S30 of driving the sample liquid to flow into the reaction pool 122 and before the step S60 of controlling the sample liquid driving apparatus 40 to periodically and repeatedly execute a second liquid pushing and drawing operation, the control method according to the present invention further includes:
step S51: switching the microfluidic biochip 10 to a state where the sample inlet thereof is separated from the sample liquid; and
step S52: controlling the sample liquid driving apparatus 40 to execute a third liquid drawing action for promoting the sample liquid to flow towards the reaction pool 122, such that at least part of the sample liquid in the micro-channel connected between the reaction pool 122 and the sample inlet 111 flows into the reaction pool 122, thus forming a preset space margin in the micro-channel, the preset space margin being used for accommodating the sample liquid pushed out by the sample liquid driving apparatus 40 when the sample liquid driving apparatus 40 executes the second liquid pushing action.

That is, after the sample liquid enters the reaction pool 122, the sample liquid driving apparatus 40 first executes a liquid drawing action (i.e., the third liquid drawing action), such that at least part of the sample liquid in the micro-channel connected between the reaction pool and the sample inlet flows into the reaction pool 122, thus forming the preset space margin in the micro-channel, the preset space margin being used for accommodating the sample liquid pushed out by the sample liquid driving apparatus 40 when the sample liquid driving apparatus 40 executes the second liquid pushing action. Thus, the problem of contamination due to a small quantity of the sample liquid being pushed out from the sample inlet 111 when the sample liquid driving apparatus 40 performs the second liquid pushing action can be avoided.

Certainly, in other embodiments, the control method according to the present invention may not include step S52, and it is only required to perform the second liquid drawing action first and then the second liquid pushing action when the sample liquid driving apparatus 40 performs the second liquid pushing and drawing operation. Thus, the preset space margin can also be reserved in the micro-channel between the reaction pool and the sample inlet, thus avoiding the problem of contamination due to a small quantity of the sample liquid being pushed out from the sample inlet 111 when the sample liquid driving apparatus 40 performs the second liquid pushing action.

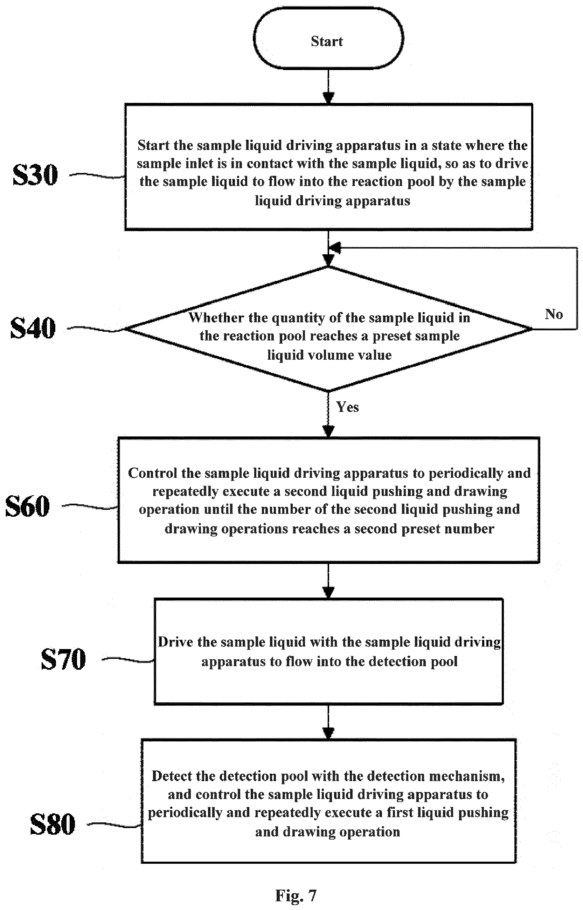

Fig. 7 is a schematic flow diagram of the control method for a microfluidic detection system according to a fifth embodiment of the present invention. Referring to Fig. 7, in some embodiments, after the step S30 of driving the sample liquid to flow into the reaction pool 122 and before the step S60 of controlling the sample liquid driving apparatus 40 to periodically and repeatedly execute a second liquid pushing and drawing operation, the control method further includes:
step S40: judging whether the quantity of the sample liquid in the reaction pool 122 reaches a preset sample liquid volume value; and
if yes, proceeding to step S60 of controlling the sample liquid driving apparatus 40 to periodically and repeatedly execute the second liquid pushing and drawing operation.

That is, in the present application, the quantity of the sample liquid entering the reaction pool 122 is accurately detected and controlled to be matched with the quantities of the reaction reagent and the detection reagent in the chip, thus avoiding the influence on the detection result caused by too much or too little sample liquid, and further improving the accuracy of the detection result.

In some embodiments, the sample liquid driving apparatus 40 may form a negative pressure in the main channel of the microfluidic biochip 10 by pumping air outwards, such that the sample liquid in contact with the sample inlet 111 enters the main channel under the action of the negative pressure. Specifically, the sample liquid driving apparatus 40 may be a micro injection pump, and further includes a driving motor, an injector, a lead screw, a slider, a piston, or the like. The quantity of displacement of the piston within the injector is positively correlated to the quantity of the sample liquid entering the microfluidic biochip 10. Therefore, the quantity of the sample liquid entering the microfluidic biochip 10 can be determined by detecting the position of the piston by a position sensor.

In these embodiments, the step S40 of judging whether the quantity of the sample liquid in the reaction pool 122 reaches a preset sample liquid volume value may specifically include:
judging whether a trigger signal for indicating that the piston of the sample liquid driving apparatus 40 moves to a preset position is received; and
if yes, determining that the quantity of the sample liquid in the reaction pool 122 reaches the preset sample liquid volume value; and if no, considering that the quantity of the sample liquid in the reaction pool 122 does not reach the preset sample liquid volume value.

It may be understood that, in some embodiments, steps S51 and S52 of the method shown in Fig. 6 may also be included in the control method shown in Fig. 7. Specifically, steps S51 and S52 may occur between steps S40 and S60.

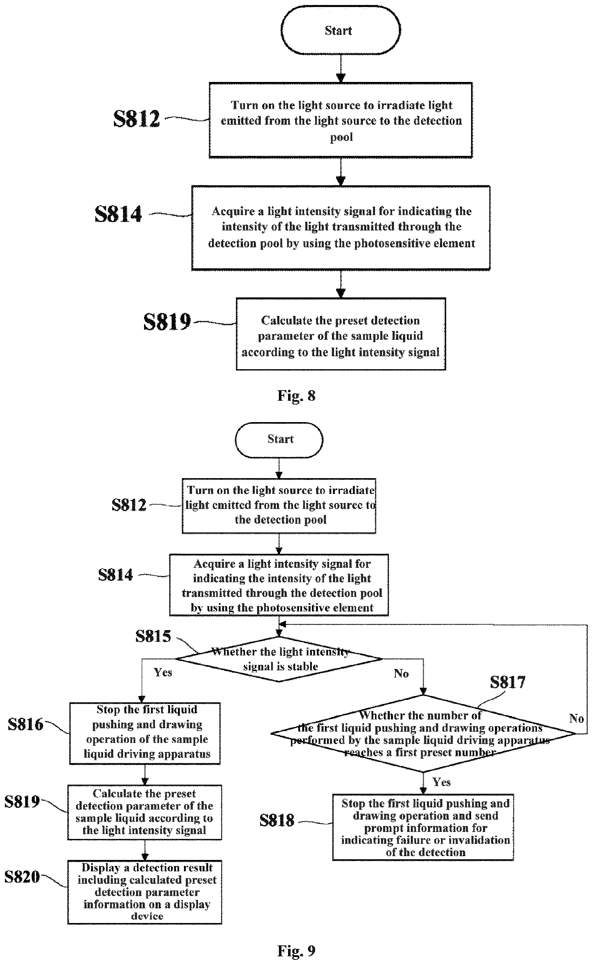

Fig. 8 is a schematic flow diagram of detection of the detection pool by the detection mechanism in an embodiment of the present invention. In some embodiments, the detection mechanism 20 may include a light source and a photosensitive element arranged on two opposite sides of the detection pool 121 respectively; the step of detecting the detection pool 121 with the detection mechanism 20 includes:
step S812: turning on the light source to irradiate light emitted from the light source to the detection pool 121;
step S814: acquiring a light intensity signal for indicating the intensity of the light transmitted through the detection pool 121 by using the photosensitive element; and
step S819: calculating the preset detection parameter of the sample liquid according to the light intensity signal.

Taking the detection of the pesticide residue parameter of the sample liquid by the microfluidic detection system 1 as an example, the reaction reagent held in the reaction pool 122 may be an enzyme reagent, and the detection reagent held in the detection pool 121 may be a color developing agent. After the sample liquid enters the reaction pool 122, pesticide residues in the sample liquid react with the enzyme reagent using the principle that a pesticide may inhibit the activity of enzyme. The solution after the reaction enters the detection pool 121. The light emitted from the light source 21 is irradiated to the detection pool 121, and the light transmitted through the detection pool 121 is introduced into the photosensitive element 22, which facilitates judgment of the change in an absorbance in the detection pool 121 using the light intensity signal received by the photosensitive element, and then facilitates calculation of a pesticide residue inhibition rate.

Fig. 9 is a schematic flow diagram of the detection of the detection pool by the detection mechanism in another embodiment of the present invention. Further, after the step S814 of acquiring a light intensity signal and before the step S818 of calculating the preset detection parameter of the sample liquid according to the light intensity signal, the control method according to the present application further includes:
step S815: judging whether the light intensity signal is stable; if yes, proceeding to step S816, and if no, proceeding to step S817;
step S816: stopping the first liquid pushing and drawing operation of the sample liquid driving apparatus 40, and proceeding to step S819;
step S817: judging whether the number of the first liquid pushing and drawing operations performed by the sample liquid driving apparatus 40 reaches a first preset number; if yes, proceeding to step S818; if no, proceeding to step S815 to continuously judge the stability of the light intensity signal; and
step S818: stopping the first liquid pushing and drawing operation and sending prompt information for indicating failure or invalidation of the detection.

It may be understood that the relatively stable light intensity signal can be received only after the sample liquid and the detection reagent fully react, and the preset detection parameter of the sample liquid calculated according to the stable light intensity signal is relatively accurate. Therefore, whether the sample liquid and the detection reagent fully react can be judged by judging whether the light intensity signal is stable. If the sample liquid and the detection reagent fully react, the sample liquid driving apparatus 40 is not required to go on performing the first liquid pushing and drawing operation, and the first liquid pushing and drawing operation may be stopped in time to reduce energy consumption. If the relatively stable light intensity signal is not received after the number of the first liquid pushing and drawing operations reaches the first preset number, the sample liquid and the detection reagent may not fully react, for example, the detection reagent loses efficacy or other reasons occur; at this point, timely stopping of the first liquid pushing and drawing operation may reduce energy consumption, prompt information is sent to remind the user that the detection is invalid or fails, and the user can conveniently make corresponding measures in time.

In one embodiment, the control method according to the present invention further includes:
after receiving a trigger signal for instructing starting of the detection function of the microfluidic detection system, starting a heating module of the microfluidic detection system, and heating the detection pool 121 by the heating module;
acquiring the temperature of the detection pool 121 periodically or in real time; and
stopping the heating module when the temperature of the detection pool 121 is higher than a preset upper temperature limit value, and restarting the heating module when the temperature in the detection pool 121 is lower than a preset lower temperature limit value.

Thus, the detection pool 121 may be guaranteed to always have a relatively constant temperature before the detection operation is performed, thus facilitating the sufficient reaction of the sample liquid and the detection reagent. Specifically, the trigger signal for instructing the starting of the detection function of the microfluidic detection system may be an electrifying signal of the microfluidic detection system, or a starting signal received by a main switch of the microfluidic detection system for starting the detection function thereof.

In some embodiments, after stopping the first liquid pushing and drawing operation of the sample liquid driving apparatus 40, the control method according to the present invention further includes:
turning off the light source and stopping the heating module. This step may occur before or after step S819.

It should be noted that the starting operation of the heating module, the temperature acquisition of the heating module, and the temperature control of the heating module are continuously performed during the whole detection process, so as to ensure that the detection pool 121 has a relatively constant temperature range all the time during the whole detection process.

In some embodiments, after the step S819 of calculating the preset detection parameter of the sample liquid according to the light intensity signal, the control method according to the present invention further includes:
step S820: displaying a detection result including calculated preset detection parameter information on a display device. The display device may be, for example, a display screen, or a color indicator lamp, or include both a display screen and a color indicator lamp.

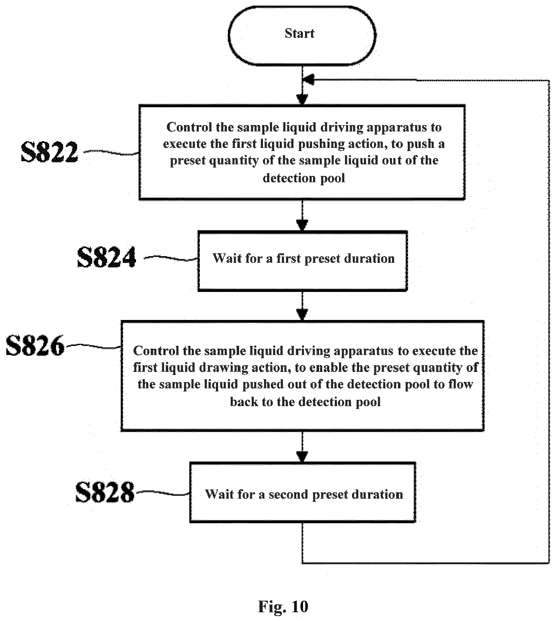

Fig. 10 is a schematic flow diagram of controlling the sample liquid driving apparatus to periodically and repeatedly execute the first liquid pushing and drawing operation in an embodiment of the present invention. In some embodiments, controlling the sample liquid driving apparatus 40 to periodically and repeatedly execute the first liquid pushing and drawing operation specifically includes:
step S822: controlling the sample liquid driving apparatus 40 to execute the first liquid pushing action, to push a preset quantity of the sample liquid out of the detection pool 121;
step S824: waiting for a first preset duration;
step S826: controlling the sample liquid driving apparatus 40 to execute the first liquid drawing action, to enable the preset quantity of the sample liquid pushed out of the detection pool 121 to flow back to the detection pool 121; and
step S828: waiting for a second preset duration, and then proceeding to step S822.

Specifically, the quantity of the sample liquid pushed out by the first liquid pushing action and the quantity of the sample liquid drawn in by the first liquid drawing action can be monitored and controlled by the displacement quantity of the piston of the sample liquid driving apparatus 40 in the injector thereof. For example, the pushed-out sample liquid is considered to reach a preset quantity when the piston moves downwards by a preset distance in the injector, and the drawn-in sample liquid is considered to reach a preset quantity when the piston moves upwards by a preset distance in the injector. The first preset duration and the second preset duration may be equal to or different from each other. After each liquid pushing action or liquid drawing action is executed, the next action is executed after a period of time, which may give the time for full mixture and reaction of the sample liquid and the detection reagent.

It should be noted that the specific process of controlling the sample liquid driving apparatus 40 to periodically and repeatedly execute the second liquid pushing and drawing operation is quite similar to the process of controlling the sample liquid driving apparatus 40 to periodically and repeatedly perform the first liquid pushing and drawing operation, and therefore is not repeated here.

Before the microfluidic biochip 10 is detected, the sample liquid is required to be added to the microfluidic biochip 10; that is, the control method according to the present invention further includes a sample loading part, which is specifically described below.

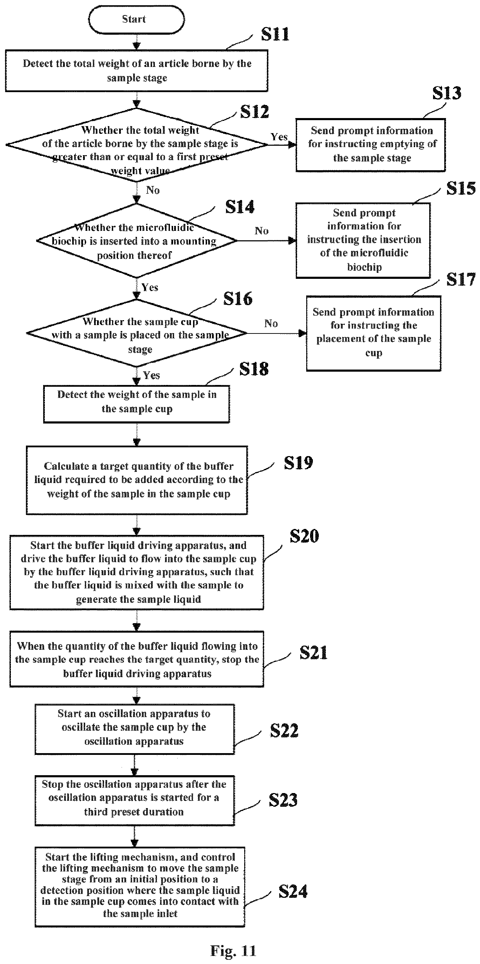

Fig. 11 is a schematic flow diagram of a sample loading process of the microfluidic detection system according to an embodiment of the present invention. The microfluidic detection system 1 further includes a sample stage 70 for placing the sample cup, a lifting mechanism 60 for driving the sample stage 70 to move, and a buffer liquid driving apparatus 30 for driving a buffer liquid to flow into the sample cup. Referring to Fig. 11, before starting the sample liquid driving apparatus 40, the control method according to the present invention further includes:
step S11: detecting the total weight of an article borne by the sample stage 70;
step S12: judging whether the total weight of the article borne by the sample stage 70 is greater than or equal to a first preset weight value; if yes, proceeding to step S13, and if no, proceeding to step S14;
step S13: sending prompt information for instructing emptying of the sample stage 70;
step S14: judging whether the microfluidic biochip 10 is inserted into a mounting position thereof; if yes, proceeding to step S 16, and if no, proceeding to step S15;
step S15: sending prompt information for instructing the insertion of the microfluidic biochip 10;
step S16: judging whether the sample cup is placed on the sample stage 70; if yes, proceeding to step S 18, and if no, proceeding to step S17;
step S17: sending prompt information for instructing the placement of the sample cup;
step S18: detecting the weight of a sample in the sample cup;
step S19: calculating a target quantity of the buffer liquid required to be added according to the weight of the sample in the sample cup;
step S20: starting the buffer liquid driving apparatus 30, and driving the buffer liquid to flow into the sample cup by the buffer liquid driving apparatus 30, such that the buffer liquid is mixed with the sample in the sample cup to generate the sample liquid; specifically, the buffer liquid driving apparatus 30 being communicated with a buffer liquid bottle 36 to supply the buffer liquid to the buffer liquid driving apparatus 30 through the buffer liquid bottle 36;
step S21: when the quantity of the buffer liquid flowing into the sample cup reaches the target quantity, stopping the buffer liquid driving apparatus 30;
step S22: starting an oscillation apparatus to oscillate the sample cup by the oscillation apparatus;
step S23: stopping the oscillation apparatus after the oscillation apparatus is started for a third preset duration; and
step S24: starting the lifting mechanism 60, and controlling the lifting mechanism 60 to move the sample stage 70 from an initial position to a detection position where the sample liquid in the sample cup comes into contact with the sample inlet 111.

Thus, a user only needs to place the sample cup onto the sample stage, and no other operations are needed, thus, the sample loading operation is convenient, the degree of automation is high, the method is time-saving and labor-saving, and the usage experience of the user is improved. Before the sample stage 70 is controlled to move, the buffer liquid is automatically injected into the sample cup when the sample cup storing the sample is placed on the sample stage 70, and is mixed with the sample to generate the sample liquid, thus omitting a process that the user manually prepares the sample liquid, avoiding the problem that the quantity or concentration of the sample liquid is not well controlled due to manual preparation of the sample liquid, improving the accuracy of the concentration and quantity of the sample liquid, and laying a foundation for the accuracy of the detection result. In the present application, the weight of the sample may also be automatically obtained, and the buffer liquid with the target quantity is automatically calculated and output according to the weight of the sample, such that the user can conveniently take the sample at will, and the accuracy of the detection result can be guaranteed. In the present application, a to-be-detected substance on the sample is promoted to be fully dissolved into the buffer liquid by the oscillation apparatus, so as to form the sample liquid with a proper concentration, thus avoiding the problem that the detection result is inaccurate due to an over low concentration of the sample liquid. In the present application, whether the microfluidic biochip 10 is mounted is automatically detected before detecting whether the sample cup is placed on the sample stage 70, and if no, the prompt information is sent to prompt the user to mount the microfluidic biochip, such that the user can conveniently and rapidly mount the microfluidic biochip before the sample cup is placed. In the present application, whether other articles (for example, a sample cup left in previous detection or other articles placed on the sample stage by the user) exist on the sample stage is automatically detected before detecting whether the microfluidic biochip 10 is mounted, and if yes, the prompt information for emptying the sample stage is sent out, such that the user can perform operations according to the prompt information, thus improving the automation degree of the microfluidic detection system, and improving the use experience of the user.

Further, when the quantity of the sample liquid in the reaction pool 122 reaches the preset sample liquid volume value, the control method according to the present invention further includes:
controlling the lifting mechanism 60 to return the sample stage 70 to the initial position thereof.

The present invention further provides a microfluidic detection system 1 operating according to the control method according to any one of the above-mentioned embodiments.

Specifically, the microfluidic detection system 1 may include a microfluidic biochip 10 for providing detection conditions and detection environments, a sample stage 70 for placing a sample cup, a lifting mechanism 60 for driving the sample stage 70 to move, a sample liquid driving apparatus 40 for driving a sample liquid to flow, a buffer liquid driving apparatus 30 for driving a buffer liquid to flow into the sample cup, a detection mechanism 20 for performing a detection operation, and a buffer liquid bottle 36 for storing the buffer liquid. The sample stage 70 may be located below the microfluidic biochip 10, such that the sample liquid in the sample cup thereon is in contact with a sample inlet 111 located at the bottom of the microfluidic biochip 10. The lifting mechanism 60 is adjacently provided on a transverse side of the sample stage 70, so as to drive the sample stage 70 to move up and down. The buffer liquid driving apparatus 30 may be provided on one side of the microfluidic biochip 10 in the transverse direction and located above the lifting mechanism 60, the sample liquid driving apparatus 40 may be provided on the other side of the microfluidic biochip 10 in the transverse direction, and the buffer liquid bottle 36 is located on a side of the sample liquid driving apparatus 40 away from the microfluidic biochip 10. Thus, the size features of each module in the vertical direction and the transverse direction can be fully utilized, such that the layout of the modules is more compact, and the occupied space is reduced as far as possible. Moreover, the modules are only arranged side by side in the vertical direction and the transverse direction, such that the thickness of the microfluidic detection system 1 in the front and rear direction is reduced as far as possible, and the microfluidic detection system is more suitable for being integrated on a refrigerator.

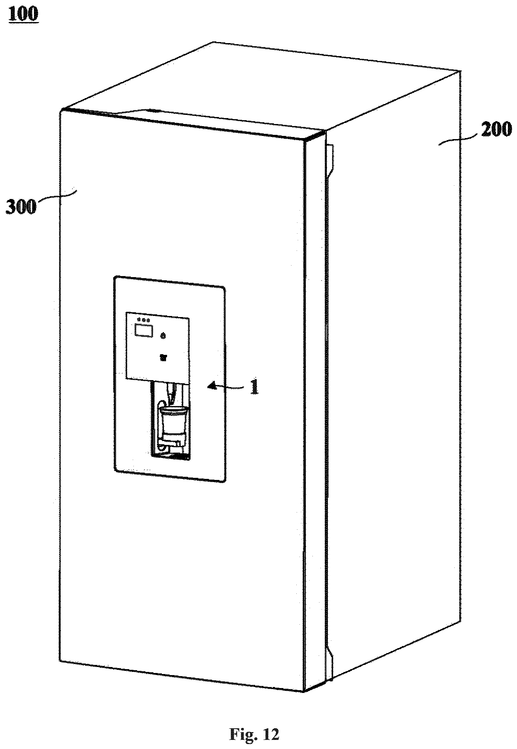

The present invention further provides a refrigerator, and Fig. 12 is a schematic structural diagram of the refrigerator according to an embodiment of the present invention; the refrigerator 100 according to the present invention includes the microfluidic detection system 1 operating according to the control method according to any one of the above embodiments, so as to integrate the microfluidic detection system 1 on the refrigerator 100. The refrigerator 100 is frequently used in daily life, and mainly configured to store food materials, and when the microfluidic detection system 1 is integrated on the refrigerator 100, a user can conveniently perform a detection operation of a food material sample by using the microfluidic detection system 1.

Further, the refrigerator 100 further includes a cabinet 200 and a door 300, the cabinet 200 defines a storage space therein, and the door 300 is connected to the cabinet 200 and configured to open and/or close the storage space. The microfluidic detection system 1 is preferably provided on the door 300, such that the operation is convenient, an original storage space in the cabinet 200 cannot be occupied, and the storage capacity of the refrigerator 100 cannot be influenced. The microfluidic detection system 1 may be electrically connected to an electrical control apparatus of the refrigerator 100, so as to provide power for the microfluidic detection system 1 by the electrical control apparatus and/or to allow signals to be transmitted between the electrical control apparatus and the microfluidic detection system 1.

The refrigerator 100 according to the present application is a refrigerator in a broad sense, and includes not only a so-called refrigerator in a narrow sense, but also a storage apparatus having a refrigerating, freezing or other storage function, for example, a refrigerating box, a freezer, or the like.

So far, those skilled in the art should be aware that, although plural exemplary embodiments of the present invention have been shown and described herein in detail, a lot of other variations or modifications conforming to the principle of the present invention can still be directly determined or derived from the contents disclosed in the present invention without departing from the protective scope as defined by the claims. Therefore, the protective scope as defined by the claims should be understood and deemed as covering all of these other variations or modifications.

## Claims

1. A control method for a microfluidic detection system (1), the microfluidic detection system (1) comprising a microfluidic biochip (10), a sample liquid driving apparatus (40) and a detection mechanism (20), the microfluidic biochip (10) being provided with a sample inlet (111) for receiving a sample liquid, a communication port (112) communicated with the sample liquid driving apparatus (40) and a detection pool (121) formed inside the microfluidic biochip (10), and the sample inlet (111), the detection pool (121) and the communication port (112) being communicated in sequence; wherein the microfluidic biochip (10) is further provided with a reaction pool (122) connected between the detection pool (121) and the sample inlet (111), and the sample inlet (111), the reaction pool (122), the detection pool (121) and the communication port (112) are sequentially communicated through a micro-channel (14);
the control method comprising:
starting the sample liquid driving apparatus (40) in a state where the sample inlet (111) is in contact with the sample liquid, so as to drive the sample liquid to flow into the reaction pool (122) by the sample liquid driving apparatus (40);
switching the microfluidic biochip (10) to a state where the sample inlet (111) thereof is separated from the sample liquid;
controlling the sample liquid driving apparatus (40) to execute a third liquid drawing action for promoting the sample liquid to flow towards the reaction pool (122), such that at least part of the sample liquid in the micro-channel (14) connected between the reaction pool (122) and the sample inlet (111) flows into the reaction pool (122), thus forming a preset space margin in the micro-channel (14), the preset space margin being used for accommodating the sample liquid pushed out by the sample liquid driving apparatus (40) when the sample liquid driving apparatus (40) executes a second liquid pushing action;
controlling the sample liquid driving apparatus (40) to periodically and repeatedly execute the second liquid pushing and drawing operation until the number of the second liquid pushing and drawing operations reaches a second preset number; the second liquid pushing and drawing operation comprising a second liquid pushing action for promoting the sample liquid to flow towards the sample inlet (111) and a second liquid drawing action for promoting the sample liquid to flow towards the reaction pool (122);
driving the sample liquid with the sample liquid driving apparatus (40) to flow into the detection pool (121);
detecting the detection pool (121) with the detection mechanism (20);
and controlling the sample liquid driving apparatus (40) to periodically and repeatedly execute a first liquid pushing and drawing operation, the first liquid pushing and drawing operation comprising a first liquid pushing action used for prompting the sample liquid to flow in the direction of the sample inlet (111), and a first liquid drawing action used for prompting the sample liquid to flow in the direction of the detection pool (121).

2. The control method according to claim 1,
wherein after driving the sample liquid to flow into the reaction pool (122) and before controlling the sample liquid driving apparatus (40) to periodically and repeatedly execute a second liquid pushing and drawing operation, the control method further comprises:
judging whether the quantity of the sample liquid in the reaction pool (122) reaches a preset sample liquid volume value; and
if yes, controlling the sample liquid driving apparatus (40) to periodically and repeatedly execute the second liquid pushing and drawing operation.

3. The control method according to claim 2,
wherein the step of judging whether the quantity of the sample liquid in the reaction pool (122) reaches a preset sample liquid volume value comprises:
judging whether a trigger signal for indicating that a piston of the sample liquid driving apparatus (40) moves to a preset position is received; and
if yes, determining that the quantity of the sample liquid in the reaction pool (122) reaches the preset sample liquid volume value.

4. The control method according to any one of claims 1 to 3, wherein the detection mechanism (20) comprises a light source and a photosensitive element arranged on two opposite sides of the detection pool (121); the step of detecting the detection pool (121) with the detection mechanism (20) comprises:
turning on the light source to irradiate light emitted from the light source to the detection pool (121);
acquiring a light intensity signal for indicating the intensity of the light transmitted through the detection pool (121) by using the photosensitive element; and
calculating a preset detection parameter of the sample liquid according to the light intensity signal.

5. The control method according to claim 4, wherein after the acquiring a light intensity signal and before the calculating a preset detection parameter of the sample liquid according to the light intensity signal, the control method further comprises:
judging whether the light intensity signal is stable;
if the light intensity signal is stable, stopping the first liquid pushing and drawing operation of the sample liquid driving apparatus (40), and calculating the preset detection parameter of the sample liquid according to the light intensity signal; if the light intensity signal is unstable, judging whether the number of the first liquid pushing and drawing operations executed by the sample liquid driving apparatus (40) reaches a first preset number; and
if yes, stopping the first liquid pushing and drawing operation and sending prompt information for indicating failure or invalidation of the detection.

6. The control method according to claim 4 or 5, further comprising:
after receiving a trigger signal for instructing starting of the detection function of the microfluidic detection system (1), starting a heating module of the microfluidic detection system (1), and heating the detection pool (121) by the heating module;
acquiring the temperature of the detection pool (121) periodically or in real time; and
stopping the heating module when the temperature of the detection pool (121) is higher than a preset upper temperature limit value, and restarting the heating module when the temperature in the detection pool (121) is lower than a preset lower temperature limit value.

7. The control method according to claim 6,
wherein after stopping the first liquid pushing and drawing operation of the sample liquid driving apparatus (40), the control method further comprises:
turning off the light source and stopping the heating module.

8. The control method according to any one of claims 1 to 7,
wherein in one and the same first liquid pushing and drawing operation, the quantity of the sample liquid pushed out by the sample liquid driving apparatus (40) performing the first liquid pushing action is the same as the quantity of the sample liquid sucked in by the sample liquid driving apparatus (40) performing the first liquid drawing action; and
in one and the same second liquid pushing and drawing operation, the quantity of the sample liquid pushed out by the sample liquid driving apparatus (40) performing the second liquid pushing action is the same as the quantity of the sample liquid sucked in by the sample liquid driving apparatus (40) performing the second liquid drawing action.

9. The control method according to any one of claims 1 to 8, wherein controlling the sample liquid driving apparatus (40) to periodically and repeatedly execute the first liquid pushing and drawing operation comprises:
controlling the sample liquid driving apparatus (40) to execute the first liquid pushing action, to push a preset quantity of the sample liquid out of the detection pool (121);
after waiting for a first preset duration, controlling the sample liquid driving apparatus (40) to execute the first liquid drawing action, to enable the sample liquid pushed out of the detection pool (121) to flow back to the detection pool (121); and
after waiting for a second preset duration, controlling the sample liquid driving apparatus (40) to execute the first liquid pushing action again, and repeating the process.

10. A microfluidic detection system (1) operating according to the control method according to any one of claims 1 to 9.

11. A refrigerator (100) comprising a microfluidic detection system (1) operating according to the control method according to any one of claims 1 to 9.

## Patentansprüche

1. Steuerverfahren für ein mikrofluidisches Detektionssystem (1), wobei das mikrofluidische Detektionssystem (1) einen mikrofluidischen Biochip (10), eine Probenflüssigkeits-Antriebsvorrichtung (40) und einen Detektionsmechanismus (20) umfasst, wobei der mikrofluidische Biochip (10) mit einem Probeneinlass (111) zur Aufnahme einer Probenflüssigkeit, einem Kommunikationsanschluss (112), der mit der Probenflüssigkeits-Antriebsvorrichtung (40) in Verbindung steht, und einem Detektionspool (121), das innerhalb des mikrofluidischen Biochips (10) ausgebildet ist, wobei der Probeneinlass (111), der Detektionspool (121) und der Kommunikationsanschluss (112) nacheinander miteinander verbunden werden; wobei der mikrofluidische Biochip (10) weiterhin mit einem Reaktionspool (122) versehen ist, der zwischen dem Detektionspool (121) und dem Probeneinlass (111) angeschlossen ist, wobei der Probeneinlass (111), der Reaktionspool (122), der Detektionspool (121) und der Kommunikationsanschluss (112) nacheinander über einen Mikrokanal (14) kommuniziert werden;
wobei das Steuerverfahren Folgendes umfasst:
Starten der Probenflüssigkeits-Antriebsvorrichtung (40) in einem Zustand, in dem der Probeneinlass (111) in Kontakt mit der Probenflüssigkeit ist, um die Probenflüssigkeit durch die Probenflüssigkeits-Antriebsvorrichtung ( 40) so anzutreiben, dass sie in das Reaktionspool (122) fließt;
Umschalten des mikrofluidischen Biochips (10) in einen Zustand, in dem der Probeneinlass (111) von der Probenflüssigkeit getrennt ist;
Steuern der Probenflüssigkeits-Antriebsvorrichtung (40), um eine dritte Flüssigkeits-Ziehaktion auszuführen, um das Fließen der Probenflüssigkeit in Richtung des Reaktionspools (122) zu unterstützen , so dass zumindest ein Teil der Probenflüssigkeit in dem Mikrokanal (14), der zwischen dem Reaktionspool (122) und dem Probeneinlass (111) verbunden ist, in das Reaktionspool (122) fließt, wodurch ein voreingestellter Raumspalt in dem Mikrokanal (14) gebildet wird, wobei der voreingestellte Raumspalt dazu verwendet wird, die Probenflüssigkeit aufzunehmen, die durch die Probenflüssigkeits-Antriebsvorrichtung (40) herausgedrückt wird, wenn die Probenflüssigkeits-Antriebsvorrichtung (40) eine zweite Flüssigkeits-Schiebeaktion ausführt;
Steuern der Probenflüssigkeits-Antriebsvorrichtung (40), um periodisch und wiederholt den zweiten Flüssigkeitsschiebe- und -ziehvorgang auszuführen, bis die Anzahl der zweiten Flüssigkeitsschiebe- und -ziehvorgänge eine zweite voreingestellte Anzahl erreicht; wobei der zweite Flüssigkeitsschiebe- und -ziehvorgang eine zweite Flüssigkeits-Schiebeaktion zum Unterstützen des Fließens der Probenflüssigkeit in Richtung des Probeneinlasses (111) und eine zweite Flüssigkeits-Ziehaktion zum Unterstützen des Fließens der Probenflüssigkeit in Richtung des Reaktionspools (122) umfasst;
Antreiben der Probenflüssigkeit mit der Probenflüssigkeits-Antriebsvorrichtung (40), damit sie in das Detektionspool (121) fließt;
Erfassen des Detektionspools (121) mit dem Detektionsmechanismus (20); und
Steuern der Probenflüssigkeits-Antriebsvorrichtung (40), um periodisch und wiederholt einen ersten Flüssigkeitsschiebe- und -ziehvorgang auszuführen, wobei der erste Flüssigkeitsschiebe- und -ziehvorgang einen ersten Flüssigkeitsschiebvorgang, der verwendet wird, um die Probenflüssigkeit zu veranlassen, in die Richtung des Probeneinlasses (111) zu fließen, und einen ersten Flüssigkeitsziehvorgang umfasst, der verwendet wird, um die Probenflüssigkeit zu veranlassen, in die Richtung des Detektionspools (121) zu fließen.

2. Steuerverfahren nach Anspruch 1,
wobei das Steuerverfahren nach dem Antreiben der Probenflüssigkeit zum Fließen in den Reaktionspool (122) und vor dem Steuern der Probenflüssigkeits-Antriebsvorrichtung (40) zum periodischen und wiederholten Ausführen eines zweiten Flüssigkeitsschiebe- und -ziehvorgangs ferner umfasst:
Beurteilen, ob die Menge der Probenflüssigkeit im Reaktionspool (122) einen voreingestellten Probenflüssigkeitsvolumenwert erreicht; und
wenn ja, Steuern der Probenflüssigkeits-Antriebsvorrichtung (40), um periodisch und wiederholt den zweiten Flüssigkeitsschiebe- und -ziehvorgang auszuführen.

3. Steuerverfahren nach Anspruch 2,
wobei der Schritt des Beurteilens, ob die Menge der Probenflüssigkeit im Reaktionspool (122) einen voreingestellten Probenflüssigkeitsvolumenwert erreicht, umfasst:
Beurteilen, ob ein Auslösesignal empfangen wird, das anzeigt, dass sich ein Kolben der Probenflüssigkeits-Antriebsvorrichtung (40) in eine voreingestellte Position bewegt; und
wenn ja, Bestimmen, dass die Menge der Probenflüssigkeit im Reaktionspool (122) einen voreingestellten Probenflüssigkeitsvolumenwert erreicht.

4. Steuerverfahren nach einem der Ansprüche 1 bis 3, wobei der Detektionsmechanismus (20) eine Lichtquelle und ein lichtempfindliches Element umfasst, die auf zwei gegenüberliegenden Seiten des Detektionspools (121) angeordnet sind; wobei der Schritt des Erfassens des Detektionspools (121) mit dem Detektionsmechanismus (20) umfasst:
Einschalten der Lichtquelle, um das von der Lichtquelle emittierte Licht auf den Detektionspool (121) zu strahlen;
Erfassen eines Lichtintensitätssignals zum Anzeigen der Intensität des durch den Detektionspool (121) übertragenen Lichts unter Verwendung des lichtempfindlichen Elements; und
Berechnen eines voreingestellten Erfassungsparameters der Probenflüssigkeit gemäß dem Lichtintensitätssignal.

5. Steuerverfahren nach Anspruch 4, wobei das Steuerverfahren nach dem Erfassen eines Lichtintensitätssignals und vor dem Berechnen eines voreingestellten Erfassungsparameters der Probenflüssigkeit gemäß dem Lichtintensitätssignal weiterhin umfasst:
Beurteilen, ob das Lichtintensitätssignal stabil ist;
wenn das Lichtintensitätssignal stabil ist, Stoppen des ersten Flüssigkeitsschiebe- und -ziehvorgangs der Probenflüssigkeits-Antriebsvorrichtung (40) und Berechnen des voreingestellten Erfassungsparameters der Probenflüssigkeit gemäß dem Lichtintensitätssignal; wenn das Lichtintensitätssignal instabil ist, Beurteilen, ob die Häufigkeit der ersten Flüssigkeitsschiebe- und -ziehvorgänge, die von der Probenflüssigkeits-Antriebsvorrichtung (40) ausgeführt werden, eine erste voreingestellte Häufigkeit erreicht; und
wenn ja, Stoppen des ersten Flüssigkeitsschiebe- und -ziehvorgangs und Senden von Informationen zur Anzeige eines Fehlers oder einer Ungültigkeit der Erkennung.

6. Steuerverfahren nach Anspruch 4 oder 5, weiterhin umfassend:
nach Empfang eines Auslösesignals zum Anweisen des Startens der Detektionsfunktion des mikrofluidischen Detektionssystems (1), Starten eines Heizmoduls des mikrofluidischen Detektionssystems (1) und Erhitzen des Detektionspools (121) durch das Heizmodul;
Erfassen der Temperatur des Detektionspools (121) periodisch oder in Echtzeit; und
Stoppen des Heizmoduls, wenn die Temperatur des Detektionspools (121) höher als ein voreingestellter oberer Temperaturgrenzwert ist, und Wiedereinschalten des Heizmoduls, wenn die Temperatur im Detektionspool (121) niedriger als ein voreingestellter unterer Temperaturgrenzwert ist.

7. Steuerverfahren nach Anspruch 6,
wobei das Steuerverfahren nach dem Stoppen des ersten Flüssigkeitsschiebe- und -ziehvorgangs der Probenflüssigkeits-Antriebsvorrichtung (40) ferner umfasst:
Ausschalten der Lichtquelle und Stoppen des Heizmoduls.

8. Steuerverfahren nach einem der Ansprüche 1 bis 7,
wobei in ein und demselben ersten Flüssigkeitsschiebe- und -ziehvorgang die Menge der Probenflüssigkeit, die durch die Probenflüssigkeits-Antriebsvorrichtung (40), die den ersten Flüssigkeits-Schiebeaktion durchführt, herausgedrückt wird, die gleiche ist wie die Menge der Probenflüssigkeit, die durch die Probenflüssigkeits-Antriebsvorrichtung (40), die den ersten Flüssigkeits-Ziehaktion durchführt, angesaugt wird; und
wobei in ein und demselben zweiten Flüssigkeitsschiebe- und -ziehvorgang die Menge der Probenflüssigkeit, die durch die Probenflüssigkeits-Antriebsvorrichtung (40), die den zweiten Flüssigkeits-Schiebeaktion durchführt, herausgedrückt wird, die gleiche ist wie die Menge der Probenflüssigkeit, die durch die Probenflüssigkeits-Antriebsvorrichtung (40), die den zweiten Flüssigkeits-Ziehaktion durchführt, angesaugt wird.

9. Steuerverfahren nach einem der Ansprüche 1 bis 8, wobei das Steuern der Probenflüssigkeits-Antriebsvorrichtung (40) zum periodischen und wiederholten Ausführen des ersten Flüssigkeitsschiebe- und -ziehvorgang Folgendes umfasst:
Steuern der Probenflüssigkeits-Antriebsvorrichtung (40), um die erste Flüssigkeits-Schiebeaktion auszuführen, um eine voreingestellte Menge der Probenflüssigkeit aus dem Detektionspool (121) zu schieben;
nach dem Warten für eine erste voreingestellte Dauer, Steuern der Probenflüssigkeits-Antriebsvorrichtung (40), um den ersten Flüssigkeits-Ziehaktion auszuführen, um zu ermöglichen, dass die Probenflüssigkeit, die aus dem Detektionspool (121) herausgedrückt wird, zurück in das Detektionspool (121) fließt; und
nach dem Warten für eine zweite voreingestellte Dauer, Steuern der Probenflüssigkeits-Antriebsvorrichtung (40), um den ersten Flüssigkeits-Schiebeaktion erneut auszuführen, und Wiederholen des Verfahrens.

10. Mikrofluidisches Detektionssystem (1), das nach dem Steuerverfahren gemäß einem der Ansprüche 1 bis 9 arbeitet.

11. Kühlschrank (100), der ein mikrofluidisches Detektionssystem (1) umfasst, das gemäß dem Steuerverfahren nach einem der Ansprüche 1 bis 9 arbeitet.

## Revendications

1. Méthode de contrôle pour un système de détection microfluidique (1), le système de détection microfluidique (1) comprenant une biopuce microfluidique (10), un appareil d'entraînement de liquide échantillon (40) et un mécanisme de détection (20), la biopuce microfluidique (10) étant pourvue d'une entrée d'échantillon (111) pour recevoir un liquide échantillon, d'un port de communication (112) communiquant avec l'appareil d'entraînement de liquide échantillon (40) et d'un pool de détection (121) formé à l'intérieur de la biopuce microfluidique (10), et l'entrée d'échantillon (111), le pool de détection (121) et le port de communication (112) étant communiqués en séquence ; dans lequel la biopuce microfluidique (10) est en outre pourvue d'un pool de réaction (122) connecté entre le pool de détection (121) et l'entrée d'échantillon (111), et l'entrée d'échantillon (111), le pool de réaction (122), le pool de détection (121) et le port de communication (112) sont communiqués séquentiellement par un micro-canal (14) ;
la méthode de contrôle comprenant :
le démarrage de l'appareil d'entraînement de liquide échantillon (40) dans un état où l'entrée d'échantillon (111) est en contact avec le liquide échantillon, de manière à faire circuler le liquide échantillon vers le pool de réaction (122) par l'appareil d'entraînement de liquide échantillon (40) ;
le passage de la biopuce microfluidique (10) à un état où son entrée d'échantillon (111) est séparée du liquide échantillon ;
la commande de l'appareil d'entraînement de liquide échantillon (40) pour exécuter une troisième action d'aspiration de liquide afin de promouvoir l'écoulement du liquide échantillon vers le pool de réaction (122), de sorte qu'au moins une partie du liquide échantillon dans le micro-canal (14) connecté entre le pool de réaction (122) et l'entrée d'échantillon (111) s'écoule dans le pool de réaction (122), formant ainsi une marge d'espace prédéfinie dans le micro-canal (14), la marge d'espace prédéfinie étant utilisée pour accueillir le liquide échantillon poussé par l'appareil d'entraînement de liquide échantillon (40) lorsque l'appareil d'entraînement de liquide échantillon (40) exécute une deuxième action de poussée de liquide ;
la commande de l'appareil d'entraînement de liquide échantillon (40) pour exécuter périodiquement et répétitivement la deuxième opération de poussée et d'aspiration de liquide jusqu'à ce que le nombre de secondes opérations de poussée et d'aspiration de liquide atteigne un deuxième nombre prédéfini ; la deuxième opération de poussée et d'aspiration de liquide comprenant une deuxième action de poussée de liquide pour promouvoir l'écoulement du liquide échantillon vers l'entrée d'échantillon (111) et une deuxième action d'aspiration de liquide pour promouvoir l'écoulement du liquide échantillon vers le pool de réaction (122) ;
l'entraînement du liquide échantillon avec l'appareil d'entraînement de liquide échantillon (40) pour l'écouler dans le pool de détection (121) ;
la détection du pool de détection (121) avec le mécanisme de détection (20) ; et
la commande de l'appareil d'entraînement de liquide échantillon (40) pour exécuter périodiquement et répétitivement une première opération de poussée et d'aspiration de liquide, la première opération de poussée et d'aspiration de liquide comprenant une première action de poussée de liquide utilisée pour inciter le liquide échantillon à s'écouler en direction de l'entrée d'échantillon (111), et une première action d'aspiration de liquide utilisée pour inciter le liquide échantillon à s'écouler en direction du pool de détection (121).

2. La méthode de contrôle selon la revendication 1,
dans laquelle après avoir fait circuler le liquide échantillon vers le pool de réaction (122) et avant de commander l'appareil d'entraînement de liquide échantillon (40) pour exécuter périodiquement et répétitivement une deuxième opération de poussée et d'aspiration de liquide, la méthode de contrôle comprend en outre :
la détermination de si la quantité de liquide échantillon dans le pool de réaction (122) atteint une valeur de volume de liquide échantillon prédéfinie ; et
si oui, la commande de l'appareil d'entraînement de liquide échantillon (40) pour exécuter périodiquement et répétitivement la deuxième opération de poussée et d'aspiration de liquide.

3. La méthode de contrôle selon la revendication 2,
dans laquelle l'étape de détermination de si la quantité de liquide échantillon dans le pool de réaction (122) atteint une valeur de volume de liquide échantillon prédéfinie comprend :
la détermination de si un signal de déclenchement indiquant qu'un piston de l'appareil d'entraînement de liquide échantillon (40) se déplace vers une position prédéfinie est reçu ; et
si oui, la détermination que la quantité de liquide échantillon dans le pool de réaction (122) atteint la valeur de volume de liquide échantillon prédéfinie.

4. La méthode de contrôle selon l'une quelconque des revendications 1 à 3, dans laquelle le mécanisme de détection (20) comprend une source de lumière et un élément photosensible disposés sur deux côtés opposés du pool de détection (121) ; l'étape de détection du pool de détection (121) avec le mécanisme de détection (20) comprenant :
l'allumage de la source de lumière pour irradier de la lumière émise par la source de lumière vers le pool de détection (121) ;
l'acquisition d'un signal d'intensité lumineuse indiquant l'intensité de la lumière transmise à travers le pool de détection (121) en utilisant l'élément photosensible ; et
le calcul d'un paramètre de détection prédéfini du liquide échantillon en fonction du signal d'intensité lumineuse.

5. La méthode de contrôle selon la revendication 4, dans laquelle après l'acquisition d'un signal d'intensité lumineuse et avant le calcul d'un paramètre de détection prédéfini du liquide échantillon en fonction du signal d'intensité lumineuse, la méthode de contrôle comprend en outre :
la détermination de si le signal d'intensité lumineuse est stable ;
si le signal d'intensité lumineuse est stable, l'arrêt de la première opération de poussée et d'aspiration de liquide de l'appareil d'entraînement de liquide échantillon (40) et le calcul du paramètre de détection prédéfini du liquide échantillon en fonction du signal d'intensité lumineuse ; si le signal d'intensité lumineuse est instable, la détermination de si le nombre de premières opérations de poussée et d'aspiration de liquide exécutées par l'appareil d'entraînement de liquide échantillon (40) atteint un premier nombre prédéfini ; et
si oui, l'arrêt de la première opération de poussée et d'aspiration de liquide et l'envoi d'une information de rappel indiquant un échec ou une invalidité de la détection.

6. La méthode de contrôle selon la revendication 4 ou 5, comprenant en outre :
après réception d'un signal de déclenchement pour ordonner le démarrage de la fonction de détection du système de détection microfluidique (1), le démarrage d'un module de chauffage du système de détection microfluidique (1) et le chauffage du pool de détection (121) par le module de chauffage ;
l'acquisition de la température du pool de détection (121) périodiquement ou en temps réel ; et
l'arrêt du module de chauffage lorsque la température du pool de détection (121) est supérieure à une valeur limite de température supérieure prédéfinie, et le redémarrage du module de chauffage lorsque la température dans le pool de détection (121) est inférieure à une valeur limite de température inférieure prédéfinie.

7. La méthode de contrôle selon la revendication 6,
dans laquelle après l'arrêt de la première opération de poussée et d'aspiration de liquide de l'appareil d'entraînement de liquide échantillon (40), la méthode de contrôle comprend en outre :
l'extinction de la source de lumière et l'arrêt du module de chauffage.

8. La méthode de contrôle selon l'une quelconque des revendications 1 à 7,
dans laquelle dans une même première opération de poussée et d'aspiration de liquide, la quantité de liquide échantillon poussée par l'appareil d'entraînement de liquide échantillon (40) effectuant la première action de poussée de liquide est la même que la quantité de liquide échantillon aspirée par l'appareil d'entraînement de liquide échantillon (40) effectuant la première action d'aspiration de liquide ; et
dans une même deuxième opération de poussée et d'aspiration de liquide, la quantité de liquide échantillon poussée par l'appareil d'entraînement de liquide échantillon (40) effectuant la deuxième action de poussée de liquide est la même que la quantité de liquide échantillon aspirée par l'appareil d'entraînement de liquide échantillon (40) effectuant la deuxième action d'aspiration de liquide.

9. La méthode de contrôle selon l'une quelconque des revendications 1 à 8, dans laquelle la commande de l'appareil d'entraînement de liquide échantillon (40) pour exécuter périodiquement et répétitivement la première opération de poussée et d'aspiration de liquide comprend :
la commande de l'appareil d'entraînement de liquide échantillon (40) pour exécuter la première action de poussée de liquide, afin de pousser une quantité prédéfinie de liquide échantillon hors du pool de détection (121) ;
après attente d'une première durée prédéfinie, la commande de l'appareil d'entraînement de liquide échantillon (40) pour exécuter la première action d'aspiration de liquide, afin de permettre au liquide échantillon poussé hors du pool de détection (121) de refluer vers le pool de détection (121) ; et
après attente d'une seconde durée prédéfinie, la commande de l'appareil d'entraînement de liquide échantillon (40) pour exécuter à nouveau la première action de poussée de liquide, et répétition du processus.

10. Un système de détection microfluidique (1) fonctionnant selon la méthode de contrôle selon l'une quelconque des revendications 1 à 9.

11. Un réfrigérateur (100) comprenant un système de détection microfluidique (1) fonctionnant selon la méthode de contrôle selon l'une quelconque des revendications 1 à 9.
